# EUROPEAN PATENT APPLICATION

(11) **EP 4 450 101 A1**
(43) Date of publication of application: **23.10.2024**
(21) Application number: 24170750.4
(22) Date of filing: 17.04.2024
(51) Int. Cl.: A61M 5/32

(54) **DEVICE FOR HOLDING A NEEDLE CAP OF A SYRINGE, AND METHOD THEREFOR**

(30) Priority: 17.04.2023 DK PA202370178; 27.09.2023 DK PA202370495
(71) Applicant: Fixum Holding ApS, 7000 Fredericia (DK)
(72) Inventor: Kjærgaard Sidelmann Nielsen, Sabina, 9700 Brønderslev (DK); Kjærgaard Sidelmann Nielsen, Dennis, 9800 Hjørring (DK)
(74) Representative: Patrade A/S

(57) **Abstract**

The present invention relates to a device (1) for holding a needle cap (10) of a syringe (11). The device comprises holding means (20) comprising at least two holding points (21) for holding the needle cap (10). The holding means (20) are configured for receiving the cap (10) when inserted along a first direction (30), applying a cap holding force exceeding a release force required to release the cap (10) from the syringe (11), and disengaging from the cap (10) when the cap is displaced along the first direction (30). The device further comprises an end stop (40) configured for withstanding a cap applying force in the first direction (30). The device further comprises fastening means (50) for releasably fastening the device to an external surface with an attachment force exceeding the applied release force and the cap applying force.

## Description

### Field of the Invention

The present invention relates to a device for holding a needle cap of a syringe. The device comprises holding means comprising at least two holding points for holding the needle cap. The holding means are configured for receiving the cap when inserted along a first direction, applying a cap holding force exceeding a release force required to release the cap from the syringe, and disengaging from the cap when the cap is displaced along the first direction. The device further comprises an end stop configured for withstanding a cap applying force in the first direction. The device further comprises fastening means for releasably fastening the device to an external surface with an attachment force exceeding the applied release force and the cap applying force.

### Background of the Invention

Syringes are widely used in the healthcare sector for example for drawing blood samples, injecting medicine, vaccinations. Handling syringes is an integral part of the daily routines for many healthcare workers while under time pressure. One of the downsides of handling needles is the inherent risk of needlestick injuries which further can cause a cross infection of blood-borne diseases such as hepatitis B and C as well as HIV.

In Denmark, the healthcare authorities recommend at least one treatment facility for needlestick injuries in each region. Upon exposure to a blood-borne disease, the injured healthcare worker will be referred to a treatment facility where the risk of infection will be accessed and preventative treatments such as medication or vaccines will be initiated.

Each needlestick injury causes a chain of reactions taking up a lot of resources, both human and financial, as well as the adverse effect to the injured healthcare worker.

Needlestick injuries often occur when the healthcare worker removes the needle cap from the syringe and especially when reapplying the needle cap to the syringe. Today, healthcare workers are advised against recapping the syringe needle to prevent the needlestick injuries, however, the routines and placement of workstations make it impossible to follow said recommendation. The needle cap often needs to be reapplied to the syringe after injecting the patient when there are no sharps containers nearby or when dosing an injectable into the syringe in a medicine room and reapplying the cap before transporting it to the patient's room.

Existing solutions often comprise screwing the cap into a cap holder thus risking unscrewing the needle unintentionally.

### Object of the Invention

It is an objective of the present disclosure to achieve a device or a method to overcome the above-mentioned drawbacks.

One objective of the present disclosure is to provide a device and method for safely releasing and holding a needle cap from a syringe, and for recapping the syringe.

A further objective is to uncap, hold, and recap syringes and needle caps of different shapes and sizes.

### Description of the Invention

One objective of the invention is achieved by a device for holding a needle cap of a syringe.

The device comprises holding means comprising at least two holding points for holding the needle cap.

The holding means are configured for:
- receiving the cap when inserted along a first direction,
- applying equal forces on the cap perpendicular to the first direction in opposite directions, and applying a holding force in the first direction exceeding a release force required to release the cap from the syringe when the syringe is moved in a direction opposite to the first direction, so that the cap is released from the syringe, and
- disengaging from the cap when the cap is displaced along the first direction.

In an aspect of the device, the holding means are configured for disengaging from the cap when the cap is displaced along the first direction and when lifted in a direction substantially perpendicular to the equal forces.

In another aspect of the device, the holding means are configured for disengaging from the cap when the cap is lifted in a direction substantially perpendicular to the equal forces.

In yet another aspect of the device, the holding means are configured for disengaging from the cap when the cap is displaced along the first direction and when displaced along a lifting direction substantially perpendicular to the first direction and the equal forces.

In a further aspect of the device, the holding means are configured for disengaging from the cap when the cap is along a lifting direction substantially perpendicular to the first direction and the equal forces.

In yet a further aspect of the device, the holding means are configured for disengaging from the cap when the cap is displaced along the direction opposite to the first direction and the equal forces do not exceed the holding force in the first direction or the release force required to release the cap from the syringe when the syringe is moved in a direction opposite to the first direction.

When the holding means applies equal forces on the cap perpendicular to the first direction in opposite directions and applies a holding force in the first direction exceeding a release force required to release the cap from the syringe, the holding means may be considered to be in an engaged position. Consequently, a holding force exceeding a release force required to release the cap from the syringe is applied in the first direction. When the holding means are in the engaged position and the syringe is moved in a direction opposite to the first direction, the syringe is released from the cap being held by the holding means.

In an aspect, the holding means may be in the engaged position when the syringe is moved in a direction opposite to the first direction and the holding means are holding the cap. The holding means may be in a passive position when the cap or capped syringe is not inserted in the device. The holding means may be in the passive position when the cap or capped syringe is held by the holding means and the syringe is; not moved, moved in the first direction, or tilted or lifted out from the in device in a direction substantially perpendicular to the equal forces.

In another aspect, the holding means may be in the engaged position when an external force is applied to the holding means. The holding means may be in the passive position when the cap or capped syringe is not inserted in the device. The holding means may be in the passive position when the cap or capped syringe is held by the holding means and the syringe is; not moved or moved in the first direction.

Applying the external force to the holding means may cause the holding points to hold the needle cap with a holding force exceeding the release force required to release the cap from the syringe when the syringe is moved in a direction opposite to the first direction.

The device furthermore comprises an end stop configured for withstanding a cap applying force in the first direction and fastening means for releasably fastening the device to an external surface with an attachment force exceeding the applied release force and the cap applying force.

'Syringe' is used to describe a syringe with a needle or a syringe wherein the needle has been disposed of. 'Syringe needle' and 'needle' will be used interchangeably to describe the removeable needle part of the syringe. The 'needle cap' or `cap' describes a removeable semirigid or rigid protection covering the syringe needle.

The use of a syringe may include injecting medicine or vaccine, dosing medicine, drawing blood, storing the syringe in between use. The different types of uses may be used interchangeably to describe any use of a syringe.

The term `healthcare worker' may be used interchangeably to describe users of the device such as nurses, biomedical laboratory technicians, doctors, social and health care workers, the patient itself, or a relative to the patient.

The device ensures safe removal of needle cap before use on a patient or dosing medicine. The healthcare professional has no direct contact with the needle cap and will as a result limit the time their hands, arms, or other body parts are in close proximity to the syringe needle after the cap is removed.

Furthermore, the device ensures a safe recapping of the needle cap to the syringe after use or between uses, again, without the healthcare worker coming into contact with the needle cap or the needle after the cap is removed.

An advantage of using the device is the prevention of needlestick injuries and thus creating a safer work environment for the healthcare worker. Furthermore, less needlestick injuries lower the costs the hospital or the employer must spend on preventative measures to limit infection and the lost work of the incapacitated healthcare worker.

The device even further ensures that the syringe can be safely transported between different workstations such as medicine room, patient's rooms, a sharps container. Additionally, the healthcare worker can dispose the needle and the needle cap in a sharps container without touching neither the needle nor the needle cap.

The device may be used to hold and/or rest the syringe in the needle cap in between uses, for instance between dosing the syringe with medicine and injecting the patient, or between using the same syringe to inject the patient in several spots.

The device may be constructed in materials suitable for medical use such as polymers or metals. The device may comprise one or more materials suitable for medical use.

The device may be reusable and cleaned in between uses. The cleaning may comprise using disinfectants, washing, autoclave, and/or sterilizing the device or part of the device.

The device may be easily transported between workstations by means of the healthcare worker carrying the device by hand or in a uniform pocket, and/or by trolly.

The fastening means may be configured for releasably mounting the device on a surface such as tabletop, a trolly, a bedframe, an IV pole, a tray, in a drawer.

The fasting means may ensure that the healthcare worker can use the device, with a single hand, to uncap and recap the syringe, as well as removing the recapped syringe from the device.

In an aspect, the fastening means may be gripping means configured for enabling the device to be handheld during use. The healthcare worker may use one hand to hold the device and the other hand to insert, uncap, recap, or lift the cap.

In another aspect, the fastening means may comprise gripping means configured for enabling the device to be used both as a handheld device and be mounted on a surface.

In yet another aspect, the fastening means may be configured for being releasably attached to the healthcare workers uniform, key hanger, or similar. By being releasably attached to the healthcare worker, the device is always within reach and can easily be removed and cleaned when necessary.

The device may be used in a clinical setting such as in a hospital or a doctor's office or in a private setting such as in a patient's home or a in a nursing home.

The end stop may be an edge on the device, or a protruding part arranged centered behind the holding means in the first direction.

In an aspect of the device, the holding means may comprise two wheels wherein each wheel comprises a holding point.

In another aspect of the device, the holding means may comprise at least one compression pin and a compression face, each comprising at least one holding point. The compression pin may be configured for applying a force to the cap in a direction towards the compression face resulting in the compression face applying an equal force to the cap in the opposite direction.

The compression pin may be constructed in a resilient material, such as silicone, rubber, thermoplastic elastomer. The compression pin may be configured for elastic deformation when the cap inserted along the first direction and/or held by the holding points. The compression pin may return to its original shape when the cap is no longer held by the holding points and is thus configured for receiving a second cap.

The compression face may be constructed in a rigid material configured for withstanding the force applied to the cap by the compression pin. The compression face may be an integrated part of the device.

An advantage of the holding means comprising the compression pin and compression face is efficient and cost-effective production.

In an aspect, the device may comprise two components;
- a main body comprising the compression face, the end stop, and the fastening means; and
- the compression pin.

The compression pin may be inserted into the main body by the healthcare worker, thus eliminating the cost of mounting during production. The compression pin may be removably inserted into the main body without the use of tools, thus enabling the parts to easily be separated and individually cleaned, reused, recycled, and replaced.

In an aspect, the compression pin may be interchangeable with compression pins of various sizes configured for holding one or more sizes of caps.

In another aspect, the compression pin may comprise a plurality of holding points, wherein the holding points are arranged with various distances to the at least one holding point arranged on the compression face. Alternatively, the device may comprise a plurality of compression pins, each with one or more holding points. The distance between the compression pin holding points and at least one compression face holding point may increase in the first direction. An advantage of the distance increasing from first direction, is that the holding means are configured for holding various sizes of needle caps. Thus, the first holding points when moving in the first direction may be configured for receiving larger cap sizes. The subsequent holding points may be configured for receiving smaller and smaller sizes of needle caps.

In yet another aspect, the device may be a monolith. An advantage of the device being a monolith is that it may be produced at a low cost and in a high quantity.

Advantageously, the device is injection molded. Preferably, the device may be injection molded in a single material. Alternatively, the device may be injection molded using two or more materials. Injection molding enables the device to be produced in high quantities at a relative low cost.

The device may be constructed in a plastic such as Polypropylene (PP), Polyethylene (PE), Polyoxymethylene (POM), etc. The plastic may preferably be made in recycled material such as recycled PP, recycled PE, recycled POM, etc.

In an aspect, the device may be used both handheld by comprising gripping means and comprise fastening means configured for enabling the device to be used both as a handheld device and be mounted on a surface.

In an aspect, the first direction may be angled relative to the external surface to ensure an optimal working posture for the healthcare worker.

In one embodiment, the device further comprises a shield arranged in front of holding means in the first direction.

A shield arranged in front of holding means in the first direction may further prevent needle stick injuries by creating a physical barrier between the hand of the healthcare worker and the syringe being inserted into the cap held in the device. The healthcare worker may hold the holding means or gripping means in one hand, which is arranged behind the shield, and hold the syringe to be uncapped, recapped, or removed in the other hand.

The shield may comprise means for fastening the device to the uniform or a key hanger of a healthcare worker.

In one embodiment of the device, the holding means apply equal forces on the cap when the holding means receives an externally applied force.

The external force may be a force applied by a healthcare worker pinching the holding means. The healthcare worker may pinch the holding means and, if applicable, the force receiver using two or more fingers to apply force on opposite sides of the holding means. In an aspect, the force receiver may be an adjustment knob and the external force may be applied by turning said adjustment knob.

An advantage of applying an external force is increasing the control of when to apply the holding force to release the cap from the syringe and when to disengage the holding means from the cap to release the cap from the holding means. Applying the external force makes the device easy to use for the healthcare workers. Applying the external force further mimics the how the healthcare workers manually would recap the syringe without using the device and thereby preventing using the device in a manner not intended.

The holding force in the first direction may only be applied to the cap when the external force is applied, thereby increasing the level of control in when the holding force exceeds the release force and thereby when the syringe can be released from the cap. It further makes it possible to better control and adjust the amount of external force applied.

In an aspect of the device, the holding means may comprise a force receiver configured for transferring an applied external force to a holding force in the first direction exceeding a release force required to release the cap from the syringe. When the holding means are in the engaged position and the syringe is moved in a direction opposite to the first direction, the cap is released from the syringe.

When the holding means are in the passive position, the force receiver may be configured for clamping onto the needle cap inserted along the first direction. The force receiver may apply a clamping force to the cap to ensure the cap is held by the holding means after the syringe is released from the cap by applying external force to the force receiver, and before the syringe is reinserted into the needle cap. The clamping force may be smaller than the holding force and the cap may disengage from holding means in the passive position when the cap or recapped syringe is displaced along the direction opposite to the first direction.

In the case of the cap being held by the force receiver, the term passive position means that no external force is applied.

The clamping force may further ensure that the holding means are configured for automatically adapting to different sizes and shapes of needle caps.

The force receiver may be flexibly connected to the holding means such that an applied external force causes the holding points to engage the needle cap with a holding force. The force receiver may be elastic deformable and decrease the distance between holding points when the external force is applied. When the external force is no longer applied or the cap is no longer held by the holding means, the force receiver may return to its original shape, pre-deformation.

An advantage of the holding means comprising the force receiver is efficient and cost-effective production.

The force receiver may be constructed by one or two slits in the holding means. The closed end of the slits may provide a spring effect to allow plastic deformation of the force receiver relative to the holding means. The slits may be smaller than the needle cap to prevent the cap, capped or the recapped syringe from exiting the holding means in a direction that is not the opposite of the first direction.

Additional safety may be achieved by limiting the direction of which the needle cap can be inserted into and removed from the device. The movement of the needle cap and the syringe may be limited to only move substantially in the first direction when inserting the needle cap in the device and inserting the syringe in the needle cap, and to only move substantially in the direction opposite to the first direction when removing the needle cap from the device and removing the syringe from the needle cap.

By eliminating the needle cap and syringe being removed from the device by being 'lifted' or 'tilted', the risk of forces accidently pushing the cap or syringe out of the device is eliminated, further increasing the safety when using the device.

In one embodiment of the device, each holding means may comprise a shaft with a rotational axis perpendicular to the first axis. The holding means may be rotationally connected to the shaft and configured for rotation around the rotational axis.

The holding means may be advantageously shaped to enable holding the needle cap when moved in the direction opposite from the first direction.

In an aspect, the holding means may be individually shaped as a circle, semi-circle, oval, crescent, triangular, or a combination.

In another aspect, the holding means may be drop-shaped with the holding points arranged on the tapered part of the drop-shape.

Rotation of the holding means may cause the cap holding space between holding means to increase or decrease depending on the direction of the rotation.

In an aspect, the holding means may rotate between 10 and 90 degrees.

In one embodiment of the device, each holding means may comprise a torsion spring configured for resetting the rotation of the holding means relative to the shaft.

When inserting a needle cap in between the at least two holding points in the first direction, the distance between the at least two holding points increases. The torsion spring may continuously attempt to reset the holding means into a position with the smallest distance between the holding points, thus holding the needle cap when it is no longer moved in the first direction.

When pulling the syringe in the direction opposite from the first direction, the distance between the at least two holding points decreases thus tightening the hold of the at least two holding points on the needle cap. As the holding force applied by the holding means when pulling the syringe exceeds the release force, the syringe is released from the cap being held in the device.

In one embodiment, the device may further comprise distance adjusting means configured for adjusting the distance between two holding means.

The distance adjusting means may comprise slits, wherein each of the holding means are displaceable within the slits.

The distance adjusting means may be configured for being in a locked position wherein the distance between the at least two holding point is fixed and in an unlocked position wherein the distance between the at least two holding points can be changed.

In an aspect of the holding means comprising shafts, the shafts may be moveable within the distance adjusting means.

The distance adjusting means may ensure that the device can adapt to needle caps of different shapes and sizes.

In one embodiment of the device, the distance adjusting means may comprise a tension spring configured for adjusting the distance between two holding means.

The tension spring may be configured for being in a taut position and/or a stationary position and/or a locked position.

In the taut position, the tension spring may continuously attempt to arrange the holding means in an innermost position in the distance adjusting means to minimize the distance between the at least two holding points.

In the stationary position, the tension spring may not affect the distance between the at least two holding points thereby enabling the distance to be adjusted.

In the locked position, the distance between the at least two holding points may be fixed.

In an aspect of the holding means comprising shafts, the tension spring may connect the shafts of the holding means.

In one embodiment of the device, the fastening means may be a suction cup and/or a clamping device or a bench screw, and/or an adhesive.

In an aspect, the fastening means may be fixed to a surface using screws or bolts.

The adhesive may be glue or double-sided tape or a sticky mat.

In an aspect, the fastening means may be the weight of the device enabling the healthcare worker to use the device with a single hand.

The suction cup may be combined with clamp or screw for tightening and releasing suction to the external surface.

The fastening means may comprise a fixture fixed to an external surface, wherein the fastening means are releasably fastened to the device.

In one embodiment of the device, the holding means may comprise barbs configured for gripping and holding the needle cap.

The barbs may ensure that the holding means have a good grip when the holding point engages the needle cap.

The barbs may comprise a ribbed surface, a tapered point, a high friction surface such as rubber, one or more indentations or notches.

A further objective of the invention is achieved by a method for releasing a needle cap from a syringe using a device according to the invention.

The method for releasing comprises acts of:
- inserting a needle cap connected to a syringe between the at least two holding points along the first direction;
- pulling the syringe in a direction opposite from the first direction causing the at least two holding points to apply equal oppositely directed forces to the cap;
- releasing the cap from the syringe by pulling with a holding force exceeding the release force required to release the cap from the syringe;
- holding the cap between the at least two holding points.

In an aspect of the method of releasing, the method may comprise pulling the syringe in a direction opposite from the first direction while the at least two holding points apply equal oppositely directed forces to the cap.

In an aspect of the method of releasing, the healthcare worker may use the device in a medicine room to remove the cap and use the un-capped syringe to dose an injectable medicine.

In another aspect of the method of releasing, the healthcare worker may use the device near the patient to remove the cap and use the syringe to draw blood, inject medicine in the patient or in an IV-bag or vaccinate the patient.

In an alternative aspect of the method, the act of pulling may comprise pulling the syringe in a direction opposite from the first direction while the at least two holding points apply equal oppositely directed forces to the cap.

In one embodiment of the method, the act of pulling may further comprises an act of applying an external force to the holding means causing the at least two holding points to apply equal oppositely directed forces to the cap.

In an alternative aspect of the method, the act of pulling comprises pulling the syringe in a direction opposite from the first direction while applying an external force to the holding means, causing the at least two holding points to apply equal oppositely directed forces to the cap.

The act of applying the external force may be performed by a healthcare worker manually applying force to the holding means, or force receiver if applicable, to achieve the holding force.

In an aspect of the holding means comprising a force receiver configured for transferring an applied external force and holding force in the first direction exceeding a release force. When the equal forces do not exceed the holding force or the release force, the force receiver may be configured for clamping onto the needle cap inserted along the first direction.

When the equal forces do not exceed the holding force or the release force, the holding means may be considered being in a passive position.

The force receiver may apply equal forces, being a clamping force, to the cap to ensure the cap is held by the holding points after the syringe is released from the cap by applying external force to the force receiver, and before the syringe is reinserted into the needle cap. The clamping force may be smaller than the holding force and the cap may disengage from holding points in the passive position then cap or recapped syringe is displaced along the direction opposite to the first direction.

A further objective of the invention is achieved by a method of recapping a needle cap from the device according to the invention.

The method of recapping comprises one or more acts of:
- reinserting the syringe in a needle cap arranged between at least two holding points along the first direction causing the at least two holding points to disengage from the cap;
- pushing the syringe in the needle cap against the end stop by applying a cap applying force in the first direction securing the cap to the syringe.

In an aspect, after dosing an injectable in a medicine room, the healthcare worker may use the device to reinsert the dosed syringe in the needle cap and transport the syringe in the device to the patient or transport the recapped syringe and/or the device to the patient. In the patient's room, the healthcare worker may perform one or more of the acts of the releasing the cap by inserting, pulling, releasing, and/or holding the cap in the device.

After using the uncapped syringe on the patient, the healthcare worker may perform the acts of the method of recapping.

Thus, the device can be used to uncap and recap the syringe with or without intermediate uncapping and recapping in between.

In another aspect, the syringe can be reinserted in the cap without recapping, wherein the cap held in the device act as a syringe holder.

In yet another aspect of recapping, the method may comprise reinserting the syringe in a needle cap arranged between at least two holding points along the first direction.

In one embodiment, the method of recapping may further comprise the act of lifting the syringe with the secured needle cap up from the device.

In one embodiment, the method of recapping may further comprise the act of disengaging from the cap when the syringe with the secured needle cap is displaced along the direction opposite to the first direction.

The recapped syringe may be lifted up from the device after being recapped or after being transported to a medicine room.

After lifting the recapped syringe up from the device, the syringe needle may be disposed of in a sharp's container.

The syringe can be lifted straight up from the device in a direction perpendicular to the first direction and the equal forces, or lifted at an angle such that the syringe is lifted in the plunger end until the cap is released from the holding means. The equal forces applied by the holding means may be insignificant in the lifting direction.

### Description of the Drawing

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation of the scope of the claimed invention. In addition, an illustrated example need not have all the aspects or advantages shown. An aspect or an advantage described in conjunction with a particular example is not necessarily limited to that example and can be practiced in any other examples even if not so illustrated, or if not so explicitly described.

Exemplary embodiments of the invention are described in the figures, wherein:
- Fig. 1: illustrates an embodiment of the device according to the invention.
- Fig. 2: illustrates distance adjusting means.
- Fig. 3: illustrates removing the cap using the device according to the invention.
- Fig. 4: illustrates reapplying the cap using the device according to the invention.
- Fig. 5: illustrates cross-sections of the device according to the invention.
- Fig. 6: illustrates holding means comprising a torsion spring.
- Fig. 7: illustrates methods of removing and reapplying the cap.
- Fig. 8: illustrates another embodiment of the device according to the invention.
- Fig. 9: illustrates removing the cap using the device according to figure 8.
- Fig. 10: illustrates reapplying the cap using the device according to figure 8.
- Fig. 11: illustrates yet another embodiment of the device according to the invention.
- Fig. 12: illustrates removing the cap using the device according to figure 11.
- Fig. 13: illustrates reapplying the cap using the device according to figure 11.
- Fig. 14: illustrates releasing the recapped syringe using the device according to figure 11.

| No. | Item |
|---|---|
| 1 | Device |
| 10 | Needle cap |
| 11 | Syringe |
| 20 | Holding means |
| 21 | Holding points |
| 22 | Equal forces |
| 23 | Shaft |
| 24 | Rotational axis |
| 25 | Torsion spring |
| 30 | First direction |
| 35 | Direction opposite the first direction |
| 40 | End stop |
| 50 | Fastening means |
| 60 | Distance adjusting means |
| 61 | Tension spring |
| 70 | Shield |
| 100 | Method of releasing |
| 200 | Inserting |
| 300 | Pulling |
| 350 | Applying |
| 400 | Releasing |
| 500 | Holding |
| 600 | Method of recapping |
| 700 | Reinserting |
| 800 | Pushing |
| 900 | Lifting |
| 950 | Disengaging |

### Detailed Description of the Invention

Exemplary examples will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects.

Throughout the specification, when an element is referred to as being "connected" to another element, the element is "directly connected" to the other element, "electrically connected", "fluidic connected" or "communicatively connected" to the other element with one or more intervening elements interposed there between.

The terminology used herein is for the purpose of describing particular examples only and is not intended to be limiting. As used herein, the terms "comprises" "comprising" "includes" and/or "including" when used in this specification specify the presence of stated features, integers, steps, operations, elements, and/or components, but do not preclude the presence or addition of one or more other features, integers, steps, operations, elements, components, and/or groups thereof.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

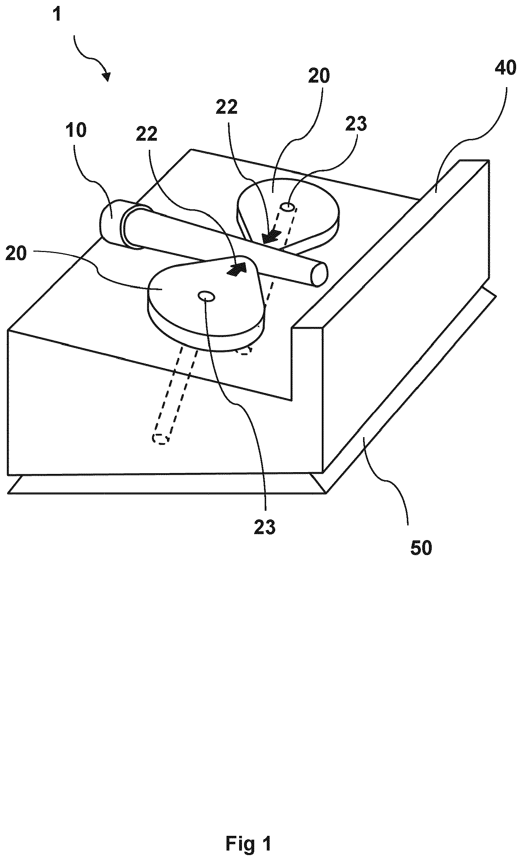

Figure 1 illustrates an embodiment of the device 1 holding a needle cap 10 of a syringe 11. The device comprises holding means 20 comprise at least two holding points 21 for holding the needle cap 10.

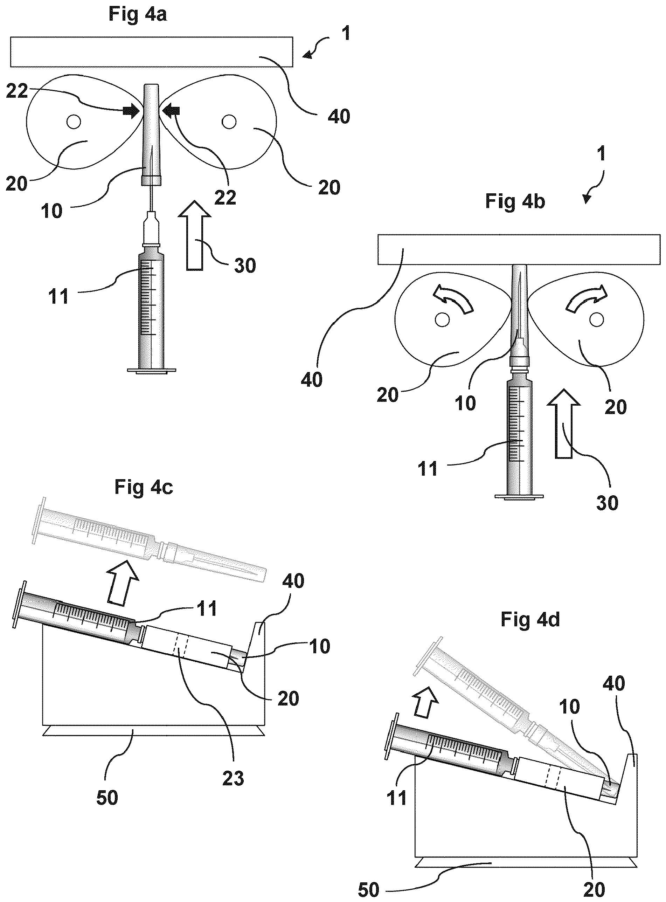

The device 1 comprises an end stop 40 configured for withstanding a cap applying force in the first direction 30, as seen on figure 4b.

The device 1 comprises fastening means 50 for releasably fastening the device to an external surface with an attachment force exceeding the applied release force and the cap applying force.

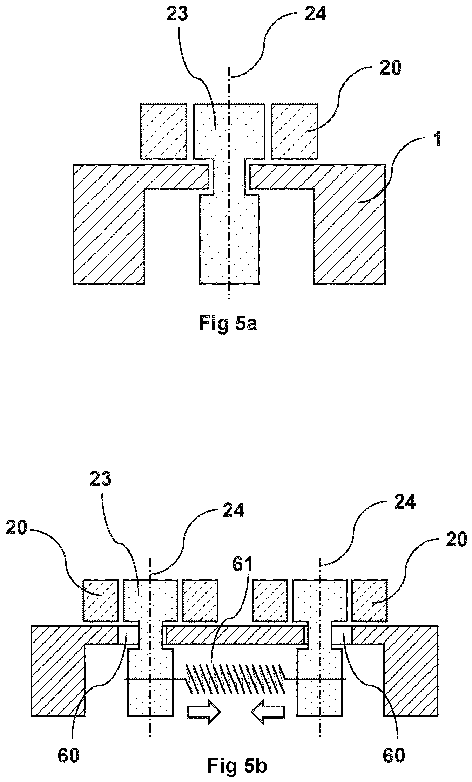

The holding means 20 further comprise a shaft 23 with a rotational axis 24 perpendicular to the first axis 30, see also figure 5a. Each of the holding means 20 are rotationally connected to a shaft 23 and configured for rotation around the rotational axis 24.

In an embodiment, the holding means 20 may comprise barbs configured for increasing the grip and hold of the needle cap 10 (not illustrated).

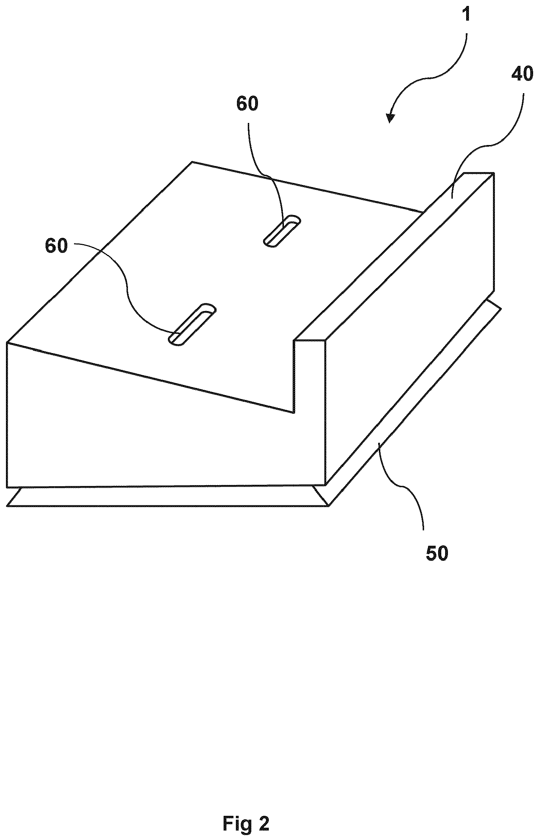

Figure 2 illustrates the device 1 further comprising distance adjusting means 60 configured for adjusting the distance between two holding means 20.

The fastening means 50 illustrated in figures 1 and 2 is a suction cup, however, other advantageous fastening means such as a suction cup, a clamping device, a bench screw, an adhesive, a weighted part may be used alone or in any combination.

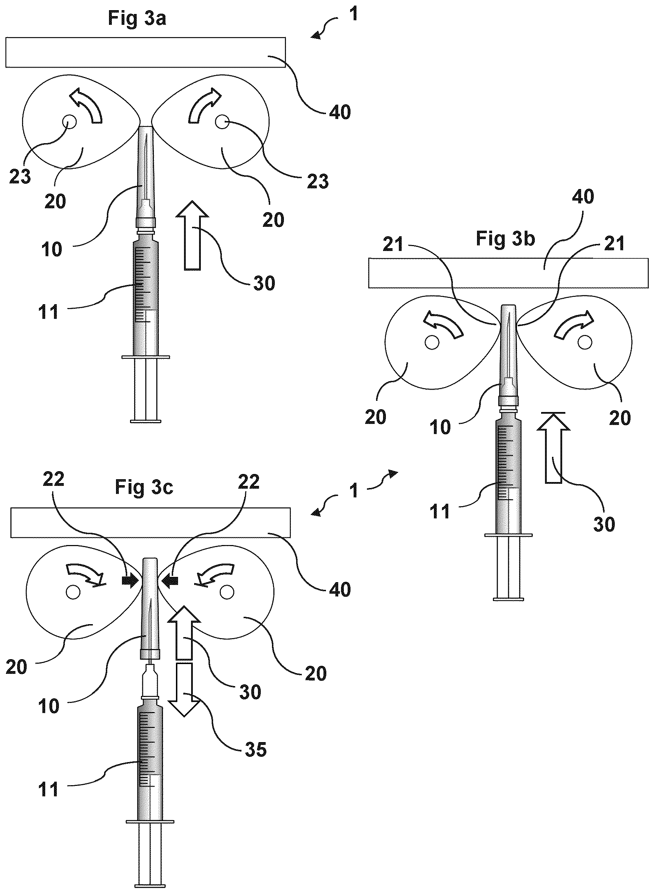

As illustrated in figures 3a and 3b, the holding means 20 are configured for receiving the cap 10 when inserted along a first direction 30. Figure 3a further illustrates an initial/reset position of the holding means before the needle cap causes the holding means to rotate in the direction of the arrows.

Figure 3c illustrates the holding means 20 being configured for applying equal forces 22 on the cap 10 perpendicular to the first direction 30 in opposite directions. When the syringe 11 is moved in a direction opposite to the first direction 35, at one point, the cap 10 prevents further rotation of the holding means 20 in the direction as indicated by the arrows. Continuously moving the syringe in the direction opposite the first direction 35 results in the holding means 20 applying a holding force in the first direction 30 exceeding a release force required to release the cap 10 from the syringe 11. As a result, the syringe is released from the cap 10 while the cap remains in the device.

Figure 4a illustrates a cap 10 being held in the device 1 by the equal forces 22. The two holding means 20 may further comprise a torsion spring 25 and/or a tension spring 61 for strengthening the hold on the cap 10. A syringe 11 is inserted needle first into the cap 10 along the first direction 30.

Figure 4b illustrates the holding means 20 being disengaged from the cap 10 when the cap 10 is displaced along the first direction 30 as the syringe 11 is inserted further into the cap 10 causing synchronized movement of the cap 10 and the syringe 11. The syringe 11 is recapped as the end of the cap 10 reaches the end stop 40 and a cap applying force is applied to the syringe 11 inserted in the cap 10.

Figures 4c and 4d illustrate the removal of the recapped syringe by lifting substantially perpendicular or at an angle.

Figure 5a illustrates a cross-sectional view of the holding means 20 comprising a shaft 23 with a rotational axis 24 perpendicular to the first axis 30. The holding means 20 are rotationally connected to the shaft 23 and configured for rotation around the rotational axis 24.

Figure 5b illustrates a cross-sectional view of the distance adjusting means 60 comprising a tension spring 61 configured for adjusting the distance between two holding means 20.

Each of the two holding means 20 illustrated in figure 5b are rotatable about the rotational axis 24 of a shaft 23. Each of the two shafts 23 are displaceable within a distance adjusting means 61 and the tension spring 61 connects the two shafts 23. The tension spring may be configured for moving the two holding means towards one another as indicated by the arrows.

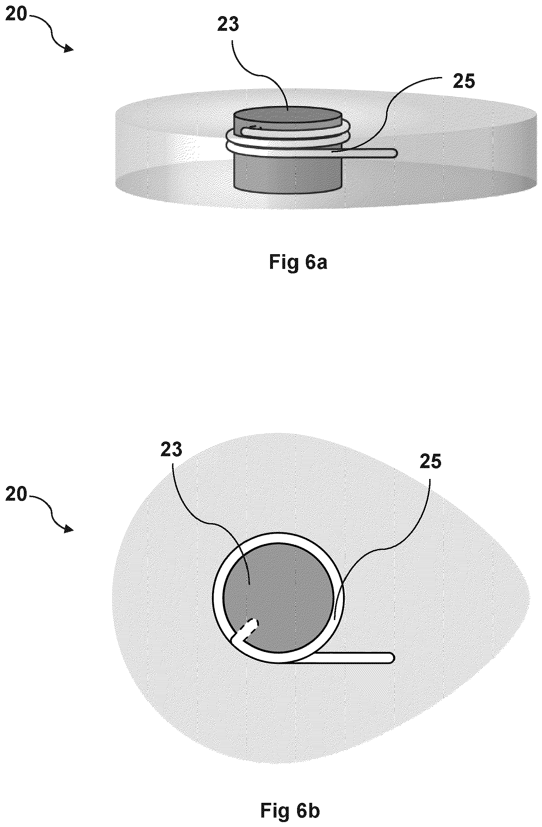

Figure 6a and 6b illustrate an embodiment of the holding means 20 comprising a torsion spring 25 configured for resetting the rotation of the holding means 20 relative to the shaft 23.

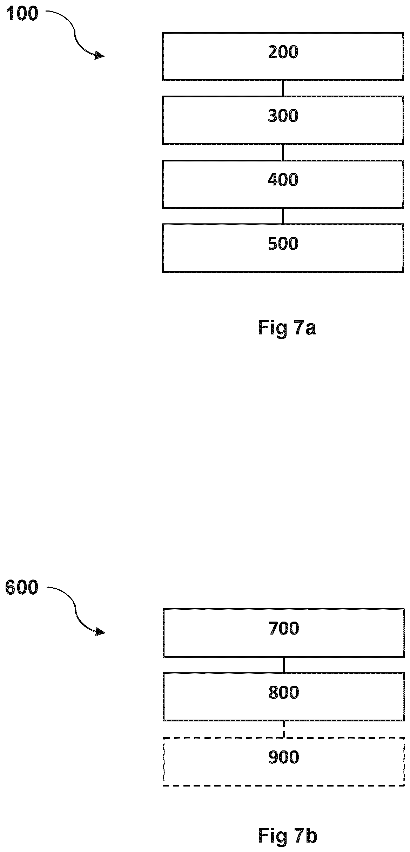

Figure 7a describes a method of releasing 100 a needle cap 10 from a syringe 11 using the device 1.

The method of releasing 100 comprises an act of inserting 200 a needle cap 10 connected to a syringe 11 between the at least two holding points 21 along the first direction 30. See figures 3a, 3b, 9a, and 12.

The method of releasing 100 comprises an act of pulling 300 the syringe 11 in a direction opposite from the first direction 35 causing the at least two holding points 21 to apply equal oppositely directed forces 22 to the cap 10.

In an aspect of the method 100, the act of releasing 100 may comprise pulling 300 the syringe 11 in a direction opposite from the first direction 35 while the at least two holding points 21 apply equal oppositely directed forces 22 to the cap 10.

The method of releasing 100 comprises an act of releasing 400 the cap 10 from the syringe 11 by pulling 300 with a holding force exceeding the release force required to release the cap 10 from the syringe 11.

The method of releasing 100 comprises an act of holding 500 the cap 10 between the at least two holding points 21.

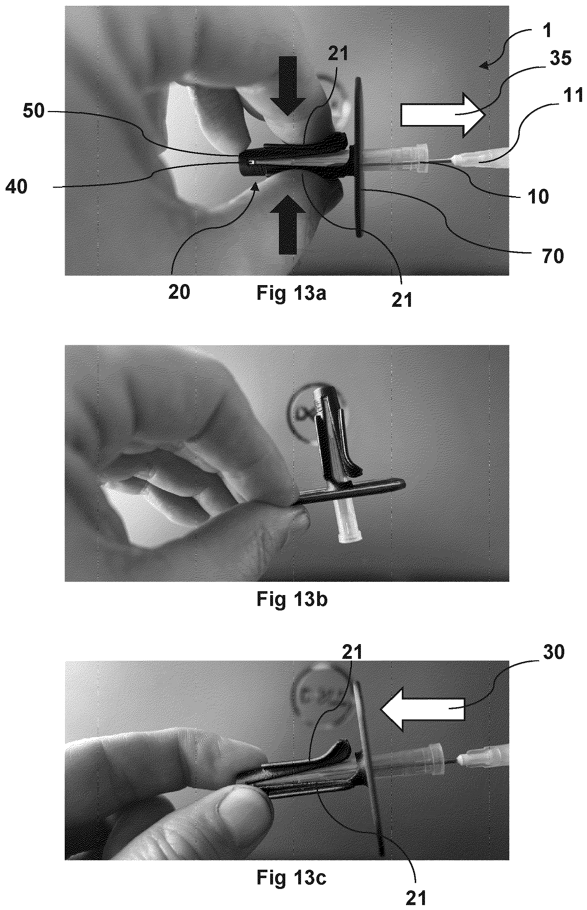

In an aspect of the method 100, the act of holding 500 may comprise holding the cap 10 between the at least two holding points 21 in the passive position. This is best illustrated in figures 13b and 13c.

In an aspect of the method of releasing 100, the act of pulling 300 may further comprise an act of applying 350 an external force to the holding means 20 to achieve the engaged position causing the at least two holding points 21 to apply equal oppositely directed forces 22 to the cap 10. This is best seen in figure 13a.

Figure 7b describes a method of recapping 600 a needle cap 10 from the device 1.

The method of recapping 600 comprises an act of reinserting 700 the syringe 11 in a needle cap 10 arranged between at least two holding points 21 along the first direction 30 causing the at least two holding points 21 to disengage from the cap 10. This is illustrated in figure 4b.

In one aspect of the method of recapping 600, the method may comprise an act of reinserting 700 the syringe 11 in a needle cap 10 arranged between at least two holding points 21 along the first direction 30 as illustrated in figure 4a, 4b, 10, and 13c.

The method of recapping 600 comprises an act of pushing 800 the syringe 11 in the needle cap 10 against the end stop 40 by applying a cap applying force in the first direction 30 securing the cap 10 to the syringe 11. This is illustrated in figures 4b,10b, and 13c.

The method of recapping 600 may further comprise the act of lifting 900 the syringe 11 with the secured needle cap 10 up from the device 1.

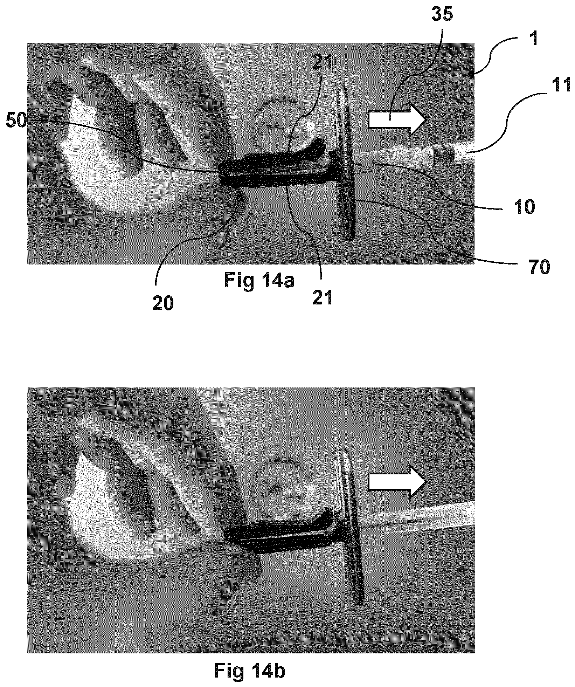

The method of recapping 600 may comprise the act of disengaging 950 from the cap 10 when the syringe 11 with the secured needle cap 10 is displaced along the direction opposite to the first direction 35, see figure 14.

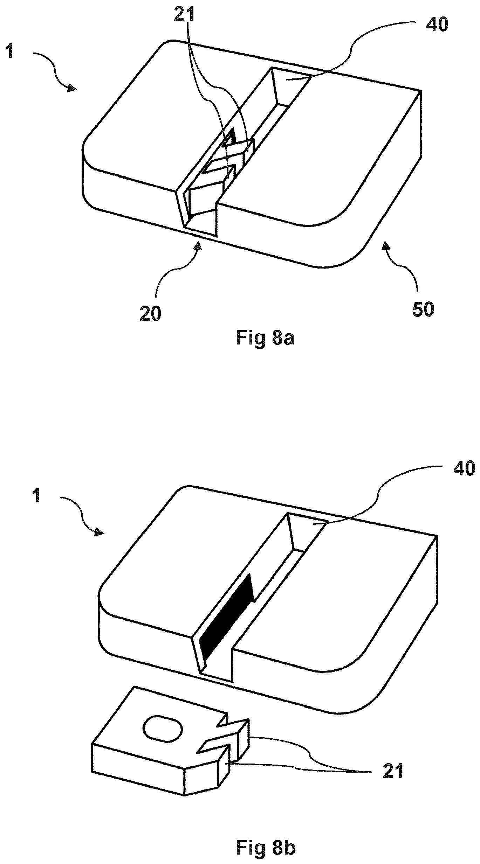

Figures 8-10 illustrate an advantageous embodiment of the invention. Figures 8a illustrate the device 1 prepared for receiving a needle cap 10 between the compression pin comprising two holding points 21 and the compression face comprising a further holding point 21 (not visible).

As illustrated in figure 8b, the device 1 may consist of two components:
- a main body comprising the compression face, the end stop 40, and the fastening means 50; and
- the compression pin.

The compression pin may be removably inserted into the main body without the use of tools. The compression pin may be inserted into the main body from a bottom side and through and at least partially through the main body. Figure 8b illustrates a fastening aperture in the compression pin configured for receiving a pin in the main body for releasably securing the compression pin in the main body.

Although not visible, the bottom of the device 1 may comprise fastening means 50 for releasably fastening the device to an external surface with an attachment force exceeding the applied release force and the cap applying force. The fastening means 50 may be a suction cup, a clamping device, a bench screw, an adhesive, a weighted part, used alone or in any combination.

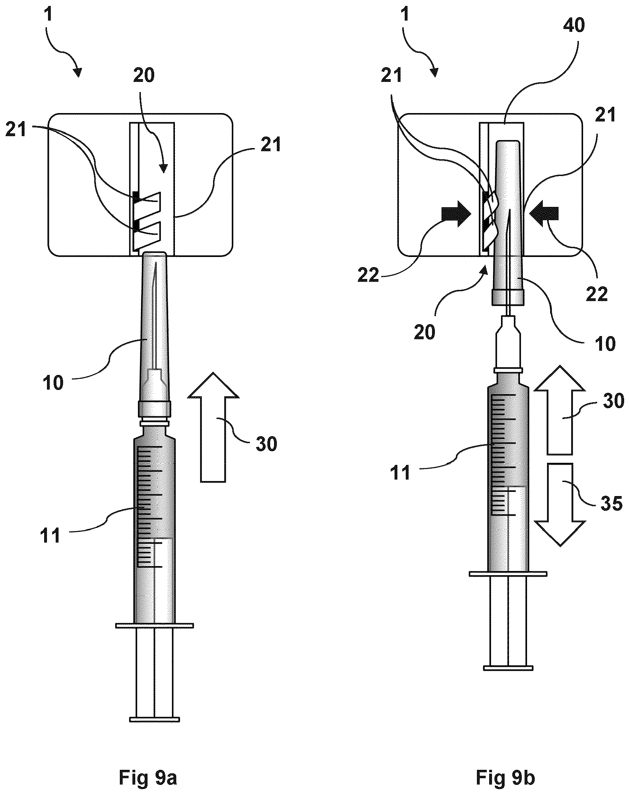

Figure 9a illustrates the device 1 holding a needle cap 10 of a syringe 11. The device comprises holding means 20 comprising three holding points 21 for holding the needle cap 10. The compression pin comprises two holding points 21 and the compression face comprises one holding point 21.

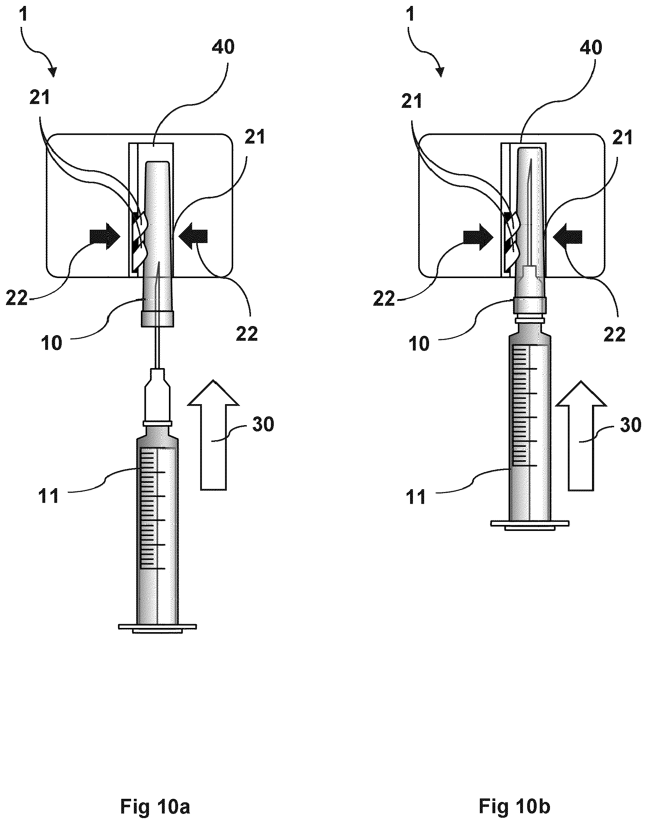

The device 1 comprises an end stop 40 configured for withstanding a cap applying force in the first direction 30, as seen on figure 10b.

In an embodiment, the holding means 20 may comprise barbs configured for increasing the grip and hold of the needle cap 10 (not illustrated).

As illustrated in figures 9a and 9b, the holding means 20 are configured for receiving the cap 10 when inserted along a first direction 30. Figure 9a further illustrates an initial/reset position of the compression pin before an inserted needle cap causes elastic deformation of the compression pin, as seen in figures 9b and 10.

Figure 9b illustrates the holding means 20 comprising the compression pin and compression face applying equal forces 22 on the cap 10 perpendicular to the first direction 30 in opposite directions. When the syringe 11 is moved in a direction opposite to the first direction 35, the cap 10 is held by the holding means, thus the holding means 20 apply a holding force in the first direction 30 exceeding a release force required to release the cap 10 from the syringe 11. As a result, the syringe is released from the cap 10 while the cap remains in the device 1.

Figure 10a illustrates a cap 10 being held in the device 1 by the equal forces 22. A syringe 11 is inserted needle first into the cap 10 along the first direction 30.

Figure 10b illustrates the cap 10 sliding in the holding means 20 when the cap 10 is displaced along the first direction 30 as the syringe 11 is inserted further into the cap 10 causing synchronized movement of the cap 10 and the syringe 11. The syringe 11 is recapped as the end of the cap 10 reaches the end stop 40 and a cap applying force is applied to the syringe 11 inserted in the cap 10.

The recapped syringe is removed from the device 1 by lifting substantially perpendicular, or at an angle, to the equal forces.

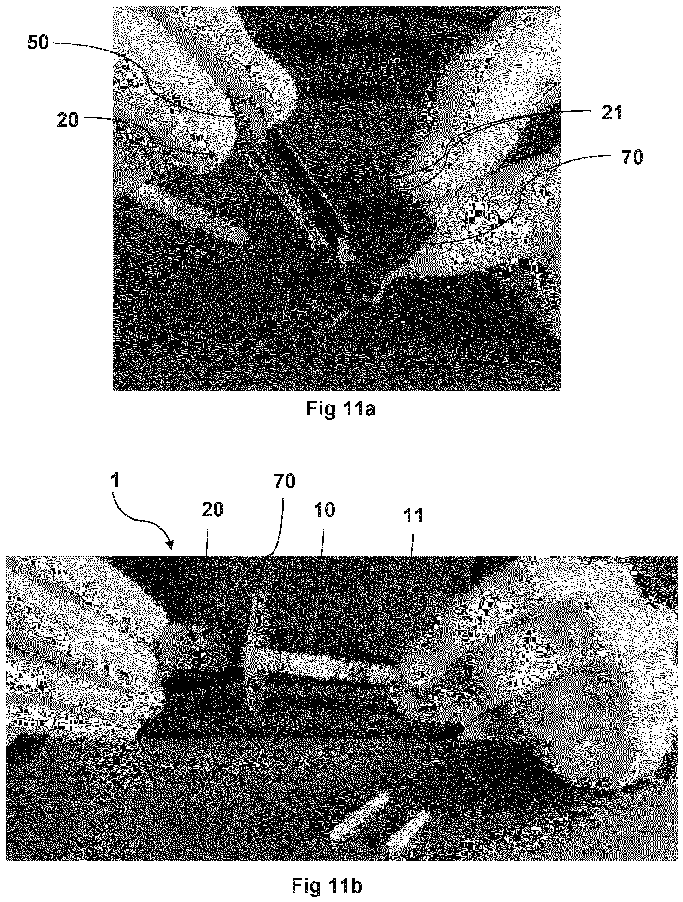

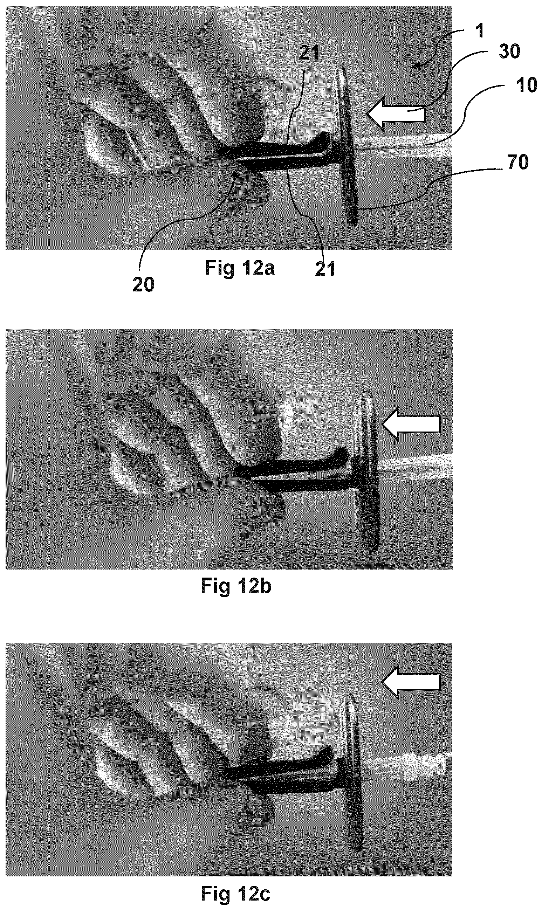

Figures 11-14 illustrate another advantageous embodiment of the invention. Figures 11a and 11b respectively illustrate the device 1 prepared for receiving a needle cap 10 between the holding points 21 and a capped syringe 10,11 inserted into the device 1. Figure 12 illustrates inserting a needle cap 10 connected to a syringe 11 between the at least two holding points 21 along the first direction 30. Figures 13a, 13b, and 13c respectively illustrate uncapping the syringe 11, the holding means 20 holding the cap 10 in the device, and the syringe 11 being recapped against the end stop 40. Figure 14 illustrates the holding means 20 disengaging from the cap 10 when the cap is displaced along the direction opposite to the first direction 30.

As illustrated, the holding means 20 comprises a force receiver configured for transferring an applied external force to a holding force in the first direction exceeding a release force required to release the cap 10 from the syringe 11. When the holding means 20 are in the engaged position and the syringe 11 is moved in a direction opposite to the first direction 35, the syringe 11 is released from the cap 10 i, see figure 13a.

As illustrated in figure 13b, when the holding means 20 are in a passive position where the equal forces 22 do not exceed the holding force and the release force, the force receiver clamps onto the needle cap 10 inserted in the device 1.

The force receiver may apply equal forces 22, being a clamping force, to the cap 10 to ensure the cap 10 is held by the holding means 20 after the syringe 11 is released from the cap 10 by applying external force to the force receiver, and before the syringe 11 is reinserted into the needle cap 10. The clamping force may be smaller than the holding force and the cap 10 may disengage from holding means 20 in the passive position when the cap 10 or recapped syringe 10,11 is displaced along the direction opposite to the first direction 35. As illustrated, the clamping force can hold the cap 10 in the device 1 when arranged upside-down.

The clamping force may further ensure that the holding means 20 are configured for automatically adapting to different sizes and shapes of needle caps 10.

The force receiver is flexibly connected to the holding means 20 such that an applied external force causes the holding points 21 to engage the needle cap 10 with a holding force. The force receiver may be elastic deformable and decrease the distance between holding points 21 when the external force is applied. When the external force is no longer applied or the cap 10 is no longer held by the holding means 20, the force receiver may return to its original shape, pre-deformation. The elastic deformation is best seen in figures 12, 13, and 14.

As illustrated in figure 13c, the syringe 11 is recapped by inserting the syringe 11 into the cap 10 in the first direction 30 and applying a cap applying force against the end stop 40.

As illustrated, the force receiver may be constructed by one or two slits in the holding means 20. The closed end of the slit(s) may provide a spring effect to allow plastic deformation of the force receiver relative to the holding means 20. The slits may be smaller than the needle cap to prevent the cap 10, capped or the recapped syringe 11 from exiting the holding means 20 in a direction that is not the opposite of the first direction 35.

The external force may be applied by a healthcare worker pinching the force receiver and on the opposite side of the holding means 20 using two or more fingers, see figure 13a.

In the illustrated embodiment, the device 1 is a monolith, as best seen in figure 11a, and is constructed in a single material.

The device comprises a shield 70 is arranged in front of holding means in the first direction 30 to further prevent needle stick injuries by creating a physical barrier between the hand of the healthcare worker and the syringe 11 being inserted into the cap 10 held in the device 1. The healthcare worker may hold the holding means 20 and/or force receiver or gripping means in one hand, all of which may be arranged behind the shield 70, and hold the syringe 11 to be uncapped, recapped, or removed in the other hand.

As illustrated in figure 11a, the shield 70 may comprise means for fastening the device 1 the uniform or a keychain of a healthcare worker.

In an aspect, the device 1 may be used both handheld by comprising gripping means and comprise fastening means 50 configured for enabling the device 1 to be used both as a handheld device and be mounted on a surface.

## Claims

1. Device (1) for holding a needle cap (10) of a syringe (11), comprising
- holding means (20) comprising at least two holding points (21) for holding the needle cap (10), said holding means (20) being configured for:
- receiving the cap (10) when inserted along a first direction (30),
- applying equal forces (22) on the cap (10) perpendicular to the first direction (30) in opposite directions, and applying a holding force in the first direction (30) exceeding a release force required to release the cap (10) from the syringe (11) when the syringe (11) is moved in a direction opposite to the first direction (35), so that the cap (10) is released from the syringe (11), and
- disengaging from the cap (10) when the cap is displaced along the first direction, a direction opposite to the first direction (30), or lifted in a direction substantially perpendicular to the equal forces (22);
- an end stop (40) configured for withstanding a cap applying force in the first direction (30).

2. Device (1) according to claim 1, further comprises fastening means (50) for releasably fastening the device to an external surface with an attachment force exceeding the applied release force and the cap applying force.

3. Device (1) according to claim 2, wherein each holding means (20) comprise a shaft (23) with a rotational axis (24) perpendicular to the first axis (30), wherein the holding means (20) are rotationally connected to the shaft (23) and configured for rotation around the rotational axis (24).

4. Device (1) according to claim 2 or 3, wherein each holding means (20) comprise a torsion spring (25) configured for resetting the rotation of the holding means (20) relative to the shaft (23).

5. Device (1) according to any of claims 2-4, further comprises distance adjusting means (60) configured for adjusting the distance between two holding means (20).

6. Device (1) according to claim 5, wherein the distance adjusting means (60) comprise a tension spring (61) configured for adjusting the distance between two holding means (20).

7. Device (1) according to any of claims 2-6, wherein the fastening means (50) is a suction cup and/or a clamping device or a bench screw, and/or an adhesive, and/or a weighted part.

8. Device (1) according to any of claims 2-7, wherein the holding means (20) comprise barbs configured for gripping and holding the needle cap (10).

9. Device (1) according to claim 1 or 2, further comprises a shield (70) arranged in front of holding means (20) in the first direction (30).

10. Device (1) according to any of claim 1, 2, or 9, wherein the holding means (20) apply equal forces (22) on the cap (10) when the holding means (20) receives an externally applied force.

11. A method (100) for releasing a needle cap (1) from a syringe using a device according to any of the preceding claims, wherein the method comprises acts of:
- inserting (200) a needle cap (10) connected to a syringe (11) between the at least two holding points (21) along the first direction (30);
- pulling (300) the syringe (11) in a direction opposite from the first direction (35) causing the at least two holding points (21) to apply equal oppositely directed forces (22) to the cap (10);
- releasing (400) the cap (10) from the syringe (11) by pulling (300) with a holding force exceeding the release force required to release the cap (10) from the syringe (11);
- holding (500) the cap (10) between the at least two holding points (21).

12. The method (100) according to claim 11, wherein the act of pulling (300) further comprises an act of applying (350) an external force to the holding means (20) causing the at least two holding points (21) to apply equal oppositely directed forces (22) to the cap (10).

13. A method of recapping (600) a needle cap (10) from the device according to claim 1-10, wherein the method comprises one or more acts of:
- reinserting (700) the syringe (11) in a needle cap (10) arranged between at least two holding points (21) along the first direction (30) causing the at least two holding points (21) to disengage from the cap (10);
- pushing (800) the syringe (11) in the needle cap (10) against the end stop (40) by applying a cap applying force in the first direction (30) securing the cap (10) to the syringe (11).

14. The method of recapping (600) according to claim 13, further comprises the act of lifting (900) the syringe (11) with the secured needle cap (10) up from the device (1).

15. The method of recapping (600) according to claim 13 or 14, further comprises the act of disengaging (950) from the cap (10) when the syringe (11) with the secured needle cap (10) is displaced along the direction opposite to the first direction (30).
